# EUROPÄISCHE PATENTANMELDUNG

(11) **EP 1 086 676 A1**
(43) Veröffentlichungstag der Anmeldung: **28.03.2001**
(21) Anmeldenummer: 00112990.7
(22) Anmeldetag: 21.06.2000
(51) Int. Cl.: A61F 13/15

(54) **Einweg-Hygieneartikel**

(30) Priorität: 23.09.1999 DE 19945548
(71) Anmelder: Christian Heinrich Sandler GmbH & Co. KG, 95126 Schwarzenbach a d Saale (DE)
(72) Erfinder:

(57) **Zusammenfassung**

Beschrieben wird ein Einweg-Hygieneartikel wie Babywindel, Trainingshöschen, Erwachseneninkontinenzhilfe, Damenhygieneartikel und dergleichen, zumindest bestehend aus einer körperseitigen flüssigkeitsdurchlässigen Abdeckung, aus einer, der Bekleidung zugewandten flüssigkeitsdichten Abdeckung und einem, zwischen diesen Schichten angeordneten absorbierenden Kern, wobei die körperseitige Abdeckung **1** eine überragende Weichheit und gute Maskierungseigenschaften aufweist und aus mindestens zwei klebemittelfrei miteinander verbundenen Schichten **2**, **3** besteht, wobei die Schichten **2**, **3** aus einer oberen perforierten Schicht **3** und einer unteren perforierten Schicht **2** bestehen, und die Perforationen **4** der oberen Schicht **3** weitgehend koaxial mit den Perforationen **8** der unteren Schicht **2** verlaufen, wobei zumindest die Perforationen **8** der unteren Schicht **2** durch einen Randbereich **5** aus geschmolzenem und wiedererstarrtem Kunststoff begrenzt sind, und die untere Schicht **2** einen Vliesstoff aus thermoplastischen Fasern umfaßt, und die obere Schicht **3** aus Mikrofasern mit einer mittleren Faserstärke von höchstens 5 µ und mit einer Flächenmasse von nicht mehr als 15 g/m² besteht.

## Beschreibung

Die Erfindung betrifft einen Einweg-Hygieneartikel, wie z. B. Babywindeln, Trainingshöschen, Erwachsenen-Inkontinenzhilfen oder Damenhygieneartikel.

Einweg-Hygieneartikel sind meist schichtenweise aufgebaut und bestehen zumindest aus einer körperseitigen, flüssigkeitsdurchlässigen Abdeckung, aus einer der Bekleidung zugewandten flüssigkeitsdichten Abdeckung und einem zwischen diesen Schichten angeordneten absorbierenden Kern.

Die körperseitige Abdeckung ist derjenige Teil des Einweg-Hygieneartikels, welcher als erstes mit den vom Körper abgegebenen Stoffen in Berührung kommt und hat deshalb die Aufgabe, die fließfähigen Bestandteile dieser Stoffe aufzunehmen und diese ins Innere des Einweg-Hygieneartikels weiterzuleiten. Zudem trägt sie dazu bei, daß einmal ins Innere des Hygieneartikels gelangte fließfähige Stoffe nicht zurück an die Körperoberfläche gelangen und dort etwa durch auftretende Benetzung der Haut z.B. Irritationen hervorrufen. Die körperseitige Abdeckung hat eine luft- und flüssigkeitsdurchlässige, poröse Struktur, deren Durchlaßvermögen durch den Einsatz von bestimmten chemischen Produkten, wie z. B. Netzmitteln weiter gesteigert werden kann.

Häufig hat eine körperseitige Abdeckung eines Einweg-Hygieneartikels nicht nur die Aufgabe, Flüssigkeiten aufzunehmen und an den absorbierenden Kern weiterzuleiten, sondern auch niedrig- bis mittelviskose Substanzen, wie zum Beispiel dünnflüssige Fäkalien. Die als körperseitige Abdeckung häufig verwendeten Vliesstoffe, sind aber wegen ihrer meist zu geringen Porengröße nicht für den Durchlaß derartiger Substanzen geeignet. Andere körperseitige Abdeckungen, wie beispielsweise perforierte Folien besitzen zwar meist größere Öffnungen, zeigen aber den Nachteil einer 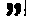 plastikartigen" Oberfläche, weshalb sie häufig von den Trägern der Hygieneartikel bzw. von deren Pflegern, abgelehnt werden.

Aus diesem Grunde werden häufig Einweg-Hygieneartikel, welche dazu bestimmt sind niedrig- bis mittelviskose Substanzen aufzunehmen, mit körperseitigen Abdeckungen ausgestattet, welche aus perforierten Vliesstoffen bestehen.

In der US-Patentschrift 5, 369, 858, wird ein mehrschichtiger Vliesstoff, bestehend aus einer Schicht textiler Fasern oder aus einem Netz polymerer Filamente als eine Schicht und aus einer Schicht schmelzgeblasener Mikrofasern vorgeschlagen. Die Perforationen, sowie die Verbindung der Schichten untereinander wird durch eine Behandlung mittels scharfer Wasserstrahlen durchgeführt. Hierbei zeigt sich der Nachteil, daß eine Behandlung von Vliesstoffen mit Wasserstrahlen in jedem Fall auch eine nachfolgende Trocknung des so behandelten Vliesstoffes erfordert. Die Trocknung von durchnäßten Vliesstoffen ist jedoch energieaufwendig und langsam, so daß sich dieses Verfahren relativ teuer und umweltbelastend darstellt.
Nachteilig wirkt sich bei einem derartigen, durch Wasserstrahlen perforierten Vliesstoff aus, daß die einzelnen Perforationen meist nicht völlig frei von unerwünschten querstehenden, die Perforationen zumindest zum Teil überspannenden Einzelfasern sind, welche durch die Verwirbelung durch die Wasserstrahlen nicht vollständig in die Ränder der Perforationen eingebunden sind. Diese unerwünschten, die Perforationen überspannenden Einzelfasern behindern das Eindringen auch von kleineren festen Bestandteilen der niedrig- bis mittelviskosen Substanzen zum Teil erheblich, weshalb derart perforierte körperseitige Abdeckungen für den genannten Einsatzzweck nur schlecht geeignet sind.
Weiterhin ist nachteilig, daß wasserstrahlperforierte körperseitige Abdeckungen keine zusätzliche Stabilisierung beispielsweise an den Perforationsrändern besitzen, was sich nachteilig auf die Gesamtfestigkeit der körperseitigen Abdeckung und auf den Zusammenhalt der Schichten auswirkt.

Die europäischen Patentschrift 587 118 B1 schlägt als Abdeckung für einen Einweg-Hygieneartikel eine perforierte Mikrofaserschicht vor, bei welcher die Perforationen dreidimensional trichterförmig in Richtung absorbierenden Kern weisen. Da ein derartiger perforierter Mikrofaservliesstoff wenig druckstabil ist, wird vorgeschlagen, diesen durch eine weitere Lage bestehend aus einer 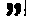 fibrösen Schicht" zu unterfüttern, wodurch eine Stabilisierung der Abdeckung gegenüber Druckeinwirkung eintritt. Die zur Unterfütterung verwendete 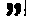 fibröse Schicht" zeigt jedoch keine Perforationen, wodurch zu erwarten ist, daß die Aufnahme von niedrig- bis mittelviskosen Substanzen erschwert wird.

Die Patentanmeldung WO 99/25911 befaßt sich mit einem Verfahren zur thermischen Perforation von Vliesstoffen. Hierbei wird die, aus thermoplastischen Fasern bestehende Vliesbahn, mittels einer beheizten Gravurwalze derart behandelt, daß an Stellen der Gravurerhebungen Perforationen im Vlies entstehen. Der an den Rändern der Perforationen angesammelte, abgeschmolzene und abgekühlte, nicht mehr faserförmige thermoplastische Kunststoff, wirkt stabilisierend und verfestigend auf den Vliesstoff, führt aber zu einer Verhärtung der Warenbahn, wie sie für den Einsatz in Hygieneprodukten nicht akzeptabel ist.
Auch tritt eine gewisse Rauhigkeit an besagten Stellen in Form von kleinen Kraterrändern und Spitzen aus abgeschmolzenen Kunststoff auf, welche besonders mit den Fingerspitzen fühlbar ist und den perforierten Vliesstoff einen 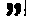 kratzigen Griff" verleiht. Derartige Stellen führen bei der Verwendung des Einweg-Hygieneartikels, verstärkt noch durch ein feuchtes Mikroklima zwischen der Haut des Trägers und dem Einweg-Hygieneartikel, leicht zu Hautirritationen.
Aus diesem Grund wird in der WO 99/25911 vorgeschlagen, zur Verringerung der 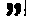 Brettigkeit" die Vliesbahn durch ein nachgeschaltetes Walzenpaar mechanisch weichzumachen, zu 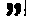 brechen".
Obwohl durch dieses Verfahren die Weichheit des perforierten Vliesstoffes deutlich erhöht wird, ist diese dennoch nicht so hoch, daß sie den Anforderungen der Träger oder deren Pfleger genügt.
Nach der Lehre der WO 99/25911 hergestellte körperseitige Abdeckungen zeigen den Vorteil, daß die einzelnen Perforationen völlig frei von unerwünschten querstehenden, die Perforationen zumindest zum Teil überspannenden Einzelfasern sind, da der hierfür verwendete Vliesstoff aus thermoplastischem Material besteht, welches unter dem Druck und der Temperatur der Erhebungen an den Kalanderwalzen vollständig abschmilzt. Es zeigen sich keine unerwünschten, die Perforationen überspannenden Einzelfasern, welche das Eindringen auch von kleineren festen Bestandteilen der niedrig- bis mittelviskosen Substanzen behindern.

Die weiterhin häufig von einer körperseitigen Abdeckung für Einweg-Hygieneprodukte geforderte Fähigkeit einer weitgehenden optischen Maskierung von, in das Einweg-Hygieneprodukt eingedrungenen, unter der körperseitigen Abdeckung abgeschiedenen niedrg- bis mittelviskose Substanzen, wird aufgrund einer gewissen Inhomogenität, der aus kardierten Vliesstoffen oder aus Spinnvliesstoffen bestehenden, nach der Lehre der WO 99/25911 hergestellten körperseitigen Abdeckung nur ungenügend erfüllt.
Die gewisse Inhomogenität dieser Vliesstoffe hat u.a. darin eine Ursache, daß die durch die genannten Verfahren verarbeitbare bzw. erzeugbare Faserfeinheit nach unten hin begrenzt ist. Diese Grenze liegt, vor dem Hintergrund einer noch wirtschaftlich durchzuführenden Fertigung, bei einer Faserfeinheit von ca. 2,0 dtex, was im Falle einer Polypropylen-Faser einem Faserdurchmesser von 16,7 µ und im Falle einer Polyester-Faser einem Faserdurchmesser von 13.6 µ entspricht.

Aufgabe der vorliegenden Erfindung ist es, eine perforierte, körperseitige Abdeckung für Einweg-Hygieneartikel mit guten Maskierungseigenschaften zur Verfügung zu stellen, welche eine überragende Weichheit aufweist. Die Aufgabe wird gemäß der Merkmale des Patentanspruches 1 gelöst. Vorteilhafte Ausgestaltungen sind in den Unteransprüchen beschrieben.

### Erläuterung der Zeichnungen

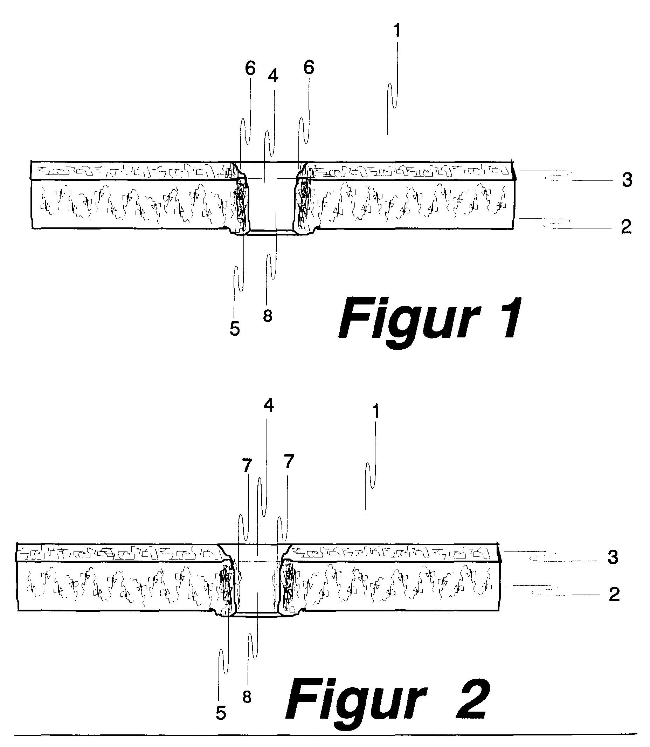

Figur 1 zeigt in vergrößerter Darstellung einen Schnitt durch eine bevorzugte Ausführungsform der erfindungsgemäßen körperseitigen Abdeckung.
Figur 2 zeigt in vergrößerter Darstellung einen Schnitt durch eine weitere bevorzugte Ausführungsform der erfindungsgemäßen körperseitigen Abdeckung.

Die erfindungsgemäße körperseitige Abdeckung **1** ist aus mindestens zwei Schichten **2**, **3** aufgebaut, wobei die untere Schicht **2** als Trägerschicht und die obere Schicht **3** als Weichmachungsschicht dient. Die Schichten besitzen Perforationen **4**, **8**. Diese Perforationen **4**, **8** dienen dazu, Körperflüssigkeiten, sowie niedrig- bis mittelviskose Substanzen aufzunehmen und dem absorbierenden Kern des Einweg-Hygieneartikels zuzuführen.

Die Perforationen **4** der oberen Schicht **3** und die Perforationen **8** der unteren Schicht **2** sind weitgehend koaxial, d. h. die körperseitige Abdeckung **1** ist durchgehend perforiert. Die Perforationen **4**, **8** haben beispielsweise einen Durchmesser von 0,5 bis 3 mm und liegen in einer Dichte von beispielsweise 0,5 bis 10 Öffnungen pro cm² vor.

Die untere Schicht **2** der körperseitigen Abdeckung **1** besteht aus einem thermisch verfestigten Vliesstoff aus thermoplastischen Fasern. Derartige Vliesstoffe können nach dem Spinnvliesverfahren oder nach dem Krempelverfahren hergestellt sein. Die thermische Verfestigung dieser Vliesstoffe kann durch eine thermomechanische Verfestigung mittels Kalanderwalze, mittels Heißluft oder durch eine Kombination der genannten Verfahren geschehen.

Der die untere Schicht **2** bildende thermisch verfestigten Vliesstoff kann eine Flächenmasse von 10 bis 100 g/m², bevorzugt von 15 bis 50 g/m² aufweisen.

Die Fasern für den thermisch verfestigten Vliesstoff können aus allen, für die thermische Verfestigung geeigneten thermoplastischen Rohstoffen, wie z.B. Polyester, Polyamid, Polyvinylalkohol, Ethylenvinylacetat usw. bestehen. In einer besonders bevorzugten Ausführungsform bestehen die Fasern aus Polyolefinen, wie z.B. Polypropylen oder Polyethylen, da diese Stoffe eine hervorragende physiologische Verträglichkeit und gute Weichheit auszeichnet.

In einer weiteren bevorzugten Ausführungsform handelt es sich bei den thermoplastischen Fasern um Fasern mit einem Bikomponenten-Aufbau. Die beiden Komponenten der Fasern können dabei innerhalb der Faser nebeneinander oder konzentrisch angeordnet sein.

Im Falle einer konzentrischen Anordnung spricht man von einer Mantel-Kern-Struktur der Faser. Hierbei ist ein Faserkern von einem Fasermantel umgeben. Der Faserkern besitzt dabei einen höheren Schmelzpunkt als der Fasermantel. Bei der Vliesverfestigung, welche zweckmäßigerweise oberhalb des Schmelzpunktes des Mantels, aber unterhalb des Schmelzpunktes des Kernes erfolgt, wird der Mantel klebrig und verfestigt den Vliesstoff, während der Kern stabil bleibt. Beispielsweise besteht der Mantel aus Polyethylen und der Kern aus Polypropylen.

Fasern bzw. Filamente, welche durch das Spinnvliesverfahren erzeugt werden, bzw. Stapelfasern, welche durch das Krempelverfahren zu Vliesstoffen verarbeitet werden, besitzen im allgemeinen eine Faserstärke von mehr als 16,7 µ, was im Falle von Polypropylenfasern einem Fasertiter von ca. 2,0 dtex entspricht. Eine Erzeugung bzw. eine Verarbeitung von Fasern mit geringerem Titer wäre mit hohem technischem Aufwand verbunden und in hohem Maße unwirtschaftlich.

Die obere Schicht **3** wird aus Mikrofasern gebildet. Diese Mikrofasern liegen bevorzugt als schmelzgeblasener Mikrofaser-Vliesstoff vor.
Schmelzgeblasene Vliesstoffe aus Mikrofasern sind Vliesstoffe, deren Herstellung z. B. im Buch 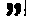 Vliesstoffe" Lünenschloß/Albrecht, Georg Thieme Verlag, Stuttgart, New York auf Seite 46 unter der Bezeichnung 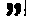 Meltblowing Prozeß" beschrieben wird. Ein nach diesem 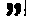 Meltblowing Prozeß" hergestellter Vliesstoff besitzt meist Faserstärken von ca. 0,5 bis 5 µ und daher eine im Vergleich zu den Stapelfaser- oder Spinnvliesstoffen eine sehr gleichmäßige Faserverteilung. Schmelzgeblasene Vliesstoffe aus Mikrofasern haben zusätzlich aufgrund der geringen Faserstärke einen sehr weichen Griff.

Die die obere Schicht **3** bildenden Mikrofasern können aus thermoplastischen Rohstoffen, wie z.B. Polyester, Polyamid, Polyvinylalkohol, Ethylenvinylacetat usw. bestehen.
In einer besonders bevorzugten Ausführungsform bestehen die Mikrofasern aus Polypropylen, da diesen Stoff eine hervorragende physiologische Verträglichkeit und gute Weichheit auszeichnet.
In der am meisten bevorzugten Ausführungsform bestehen die Míkrofasern aus Polyethylen, da diese Fasern einen extrem hohen Weichheitsgrad besitzen.

Die obere Schicht **3** aus Mikrofasern besitzt eine Flächenmasse von maximal ca. 15 g/m², da erfahrungsgemäß sonst ein inneres Spalten, und damit eine geringe Abriebfestigkeit der oberen Schicht zu befürchten ist.

In einer besonders bevorzugten Ausführungsform besitzt die obere Schicht **3** aus Mikrofasern eine Flächenmasse von weniger als 8 g/qm.

Der die untere Schicht **2** bildende thermisch verfestigte Vliesstoff ist mit den, die obere Schicht **3** bildenden Mikrofasern klebemittelfrei verbunden. Dies kann beispielsweise verfahrensmäßig dadurch realisiert werden, daß der die untere Schicht bildende thermisch verfestigte Vliesstoff mit einem in situ erzeugten schmelzgeblasenen Vliesstoff aus Mikrofasern versehen wird.
Mit 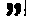 in situ erzeugt" ist gemeint, daß die durch den Meltblowing-Prozeß erzeugten Mikrofasern unmittelbar nach deren Erzeugung direkt auf dem thermisch verfestigten Vliesstoff abgelegt werden, wobei sie sich mit diesem mechanisch durch Verhakung mit abstehenden Fasern und/oder mit Unebenheiten des thermisch verfestigten Vliesstoffes verbinden. Zusätzlich kann die Verbindung über eine Restklebrigkeit der in situ erzeugten Mikrofasern mit den Fasern des thermisch verfestigten Vliesstoffes erfolgen.

In einer ersten bevorzugten Ausführungsform sind die obere Schicht **3** und die untere Schicht **2** am Randbereich **5** der Perforationen miteinander verschweißt.
(Mit Randbereich **5** im Sinne dieser Erfindung ist die Innenfläche der, die Perforationen **4**, **8** begrenzenden Wandung gemeint, welche sich von der Kante der Perforation **4** der oberen Schicht **3** bis zur Kante der Perforation **8** der unteren Schicht **2** erstreckt.)
Hierbei bildet sich ein verschweißter Bereich **6** aus, in welchem die Polymerschmelzen miteinander verschmolzen sind.
Dies kann verfahrensmäßig beispielsweise dadurch geschehen, daß die untere Schicht **2** vor dem Perforationsprozeß mit der oberen Schicht **3** versehen wird und erst danach ein Perforationsprozeß, beispielsweise wie in der DE 34 16 004 vorgeschlagen, durchgeführt wird. Bei derartiger Vorgehensweise entsteht an der Stelle der Perforation ein Randbereich **5** aus geschmolzenem und wieder erstarrtem Kunststoff sowohl an der oberen Schicht **3** wie an der unteren Schicht **2 .** Hierbei bildet sich im Bereich der Grenze zwischen oberer Schicht **3** und unterer Schicht **2** ein verschweißter Bereich **6** aus, in welchem die Polymerschmelzen der oberen Schicht **3** und unteren Schicht **2** ineinandergeflossen und miteinander verbunden sind.
Dabei bildet sich offensichtlich über dem, den Griff verhärtenden Randbereich **5** der Perforationen **8** der unteren Schicht **3**, eine feine, die Verhärtungen überdeckende Haut aus geschmolzenem Kunststoff der oberen Schicht **2** aus, welcher die vorab beschriebenen Kraterränder und Spitzen des Randbereiches **5** der Perforationen **8** der unteren Schicht **2** überdeckt. Hierbei bildet die obere Schicht **2** aufgrund ihrer geringen Flächenmasse an den Perforationen **4** einen wesentlich weniger massiven Randbereich **5** aus, als es bei den Perforationen **8** der unteren Schicht **2** der Fall ist wobei der Gesamteindruck eines weicheren Griffes entsteht.

In einer weiteren bevorzugten Ausführungsform ist die obere Schicht **3** mit der unteren Schicht **2** durch mechanische Verhakung der Mikrofasern mit dem Randbereich **5** der Perforation **8** der unteren Schicht **2** verbunden, wobei sich ein Übergangsbereich **7** bildet, in welchem sich die Mikrofasern über den Randbereich **5** der Perforation **8** der unteren Schicht **2** erstrecken. Hierbei können Mikrofasern der oberen Schicht **3** in die Perforation **8** der unteren Schicht **2** ragen, wobei sie sich mechanisch mit dem Rand verhaken.

Verfahrenstechnisch kann dies beispielsweise dadurch realisiert werden, daß ein, nach der Lehre der DE 34 16 004 perforierter und ggf. zusätzlich nach der Lehre der WO 99/25911, mechanisch weichgemachter thermisch verfestigter Vliesstoff nach dessen Perforation mit einem in situ erzeugten schmelzgeblasenen Mikrofaservliesstoff versehen wird und dessen Perforationen **4** danach beispielsweise durch Anlegen eines Vakuums koaxial mit den Perforationen **8** des die untere Schicht bildenden Vliesstoffes erzeugt werden. Hierbei wird der Mikrofaservliesstoff an den Stellen der Perforationen **8** der unteren Schicht **2** durch das Vakuum aufgerissen, wobei sich die, die Perforation **8** der unteren Schicht **2** überspannenden Mikrofasern an den Randbereich **5** der Perforation **8** legen und sich mechanisch mit diesem verhaken.

Aufgrund der die obere Schicht **3** bildenden Mikrofasern, zeigt die körperseitige Abdeckung eine überragende Weichheit, weshalb sie auch bei Einweg-Hygieneartikeln eingesetzt werden kann, welche für besonders hautempfindliche Träger vorgesehen sind. Hierbei werden auch die, die gewisse Rauhigkeit in Form von kleinen Kraterrändern und Spitzen erzeugenden Stellen aus abgeschmolzenen Kunststoff, welche an der unteren Schicht **2** vorhanden sind durch die obere Schicht **3** überdeckt, so daß diese Stellen sich weder rauh anfühlen, noch Hautirritationen verursachen können.

Die erfindungsgemäße körperseitige Abdeckung **1** zeigt aufgrund der hohen Homogenität, der aus Mikrofasern bestehenden oberen Schicht **3** eine sehr gleichmäßige Optik. Die obere Schicht **3** bewirkt aufgrund ihrer feinen Faser und ihrer Gleichmäßigkeit eine erhöhten Opazität der körperseitigen Abdeckung und ist daher in der Lage, in das Einweg-Hygieneprodukt eingedrungene, unter der körperseitigen Abdeckung abgeschiedene niedrig- bis mittelviskose Substanzen zufriedenstellend zu maskieren.

Es versteht sich von selbst, daß die erfindungsgemäße, körperseitige Abdeckung **1** je nach Bedarf, unterschiedliche zusätzliche Ausrüstungen enthalten kann.

Die die obere Schicht **3** bildenden Mikrofasern besitzen vom Polymer her meist einen hydrophoben Charakter. Dieser hydrophobe Charakter kann durch eine geeignete Ausrüstung, wie beispielsweise durch die Ausrüstung mit einem Fluorcarbonharz oder einem Silikonprodukt weiter verstärkt werden.

Alternativ kann die erfindungsgemäße körperseitige Abdeckung **1** auch hydrophil ausgerüstet sein. Um eine entsprechende Flüssigkeitsrichtung zu erreichen, ist es auch denkbar, daß sich Zonen mit hydrophilem Charakter mit Zonen mit hydrophobem Charakter abwechseln.

Desgleichen kann beispielsweise die obere Schicht **3** einen hydrophoben Charakter, die untere Schicht **2** einen hydrophilen Charakter besitzen, etwa, um auftreffende Körperflüssigkeiten möglichst schnell von der Oberseite des Hygieneartikels in dessen innere Bereiche zu transportieren.

## Patentansprüche

1. Einweg-Hygieneartikel wie Babywindel, Trainingshöschen, Erwachseneninkontinenzhilfe, Damenhygieneartikel und dergleichen, zumindest bestehend aus einer körperseitigen flüssigkeitsdurchlässigen Abdeckung, aus einer, der Bekleidung zugewandten flüssigkeitsdichten Abdeckung und einem, zwischen diesen Schichten angeordneten absorbierenden Kern, wobei die körperseitige Abdeckung **1** aus mindestens zwei klebemittelfrei miteinander verbundenen Schichten **2**, **3** besteht, wobei die Schichten **2**, **3** aus einer oberen perforierten Schicht **3** und einer unteren perforierten Schicht **2** bestehen, und die Perforationen **4** der oberen Schicht **3** weitgehend koaxial mit den Perforationen **8** der unteren Schicht **2** verlaufen, wobei zumindest die Perforationen **8** der unteren Schicht **2** durch einen Randbereich **5** aus geschmolzenem und wiedererstarrtem Kunststoff begrenzt sind, und die untere Schicht **2** einen Vliesstoff aus thermoplastischen Fasern umfaßt, und die obere Schicht **3** aus Mikrofasern mit einer mittleren Faserstärke von höchstens 5 µ und mit einer Flächenmasse von nicht mehr als 15 g/m² besteht.

2. Einweg-Hygieneartikel nach Anspruch 1
**dadurch gekennzeichnet**,
daß die obere Schicht und die untere Schicht an den Randbereichen der Perforationen miteinander verschweißt sind.

3. Einweg-Hygieneartikel nach Anspruch 1
**dadurch gekennzeichnet**,
daß Mikrofasern der oberen Schicht und der Randbereich der Perforationen der unteren Schicht mechanisch miteinander verhakt sind.
